(19) Europäisches Patentamt
European Patent Office
Office européen des brevets

(11) EP 4 059 568 A1

(12) EUROPEAN PATENT APPLICATION

(43) Date of publication:
21.09.2022 Bulletin 2022/38

(21) Application number: 21163132.0

(22) Date of filing: 17.03.2021

(51) International Patent Classification (IPC):
A61P 33/06 (2006.01)     A61K 35/17 (2015.01)
C12N 5/0783 (2010.01)

(52) Cooperative Patent Classification (CPC):
A61P 33/06; A61K 35/17; C12N 5/0646

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Schmidt-Wolf, Ingo
53127 Bonn (DE)

(72) Inventor: Schmidt-Wolf, Ingo
53127 Bonn (DE)

(74) Representative: Letzelter, Felix Phillip
Meissner Bolte Patentanwälte
Rechtsanwälte Partnerschaft mbB
Widenmayerstraße 47
80538 München (DE)

(54) **CYTOKINE-INDUCED KILLER CELLS FOR THE TREATMENT OF MALARIA**

(57)     The present invention relates to cytokine induced Killer cells (CIKs) for use in the treatment of diseases caused by a Plasmodium infection.

Figure 1

**Description**

The present invention is directed to Cytokine-Induced Killer cells (CIK), for use in the treatment of diseases caused by a Plasmodium infection.

Mankind has been suffering from diseases caused by Plasmodium infections since ages and since no vaccine against diseases caused by Plasmodium infections is known so far, the provision of medication against diseases caused by Plasmodium infections is of major importance.

The major disease caused by a Plasmodium infection is malaria, which is known in the forms of malaria tropica, malaria quartana, malaria tertiana and/or malaria quotidiana.

There is a recent hypothesis of emergence of virulent Plasmodium falciparum in Africa within the past 6000 years due to a cascade of changes in human behaviour and mosquito transmission (Deirdre et al. Science. 2003 Apr 11;300(5617):318-21). This virulence together with transmission of malaria through population inflows from highly endemic areas, which have been poorly controlled, poses great challenges in the fight against the disease by national malaria control programs (Aayush Khadka et al. Malar J (2018) 17:224). Early 2020 WHO reports showed that the global burden of malaria as of 2018 stood at an estimate of 228 million cases and an estimated number of 405 000 deaths, with children below 5 years of age being the most vulnerable group affected by the disease. Tropical African Region carries a disproportionately high share of the global malaria burden.

In 2019, ECDC's surveillance reports showed that for 2018, 8349 malaria cases were registered in the EU/EEA, with majority of cases being imported from endemic regions. German surveillance data also showed a sharp increase of malaria cases in 2014 and 2015 due to the increased arrival of refugees from malaria endemic countries (see: https://www.ecdc.europa.eu/en/publications-data/malaria-annual-epidemiologicalreport-2018 and Vygen-Bonnet et al. Changes in malaria epidemiology in Germany, 2001-2016: a time series analysis. 2018-01-15).

The unicellular protozoan parasite (Plasmodium falciparum) of humans is the deadliest species of Plasmodium that cause malaria in humans. It has been hypothesized that malarial symptoms are caused by an endogenous form of vitamin A intoxication induced by the parasites following their egress from the liver. Following this hypothesis, the parasites emerge from the liver packed with vitamin A and use retinoic acid (RA) as a cell membrane destabilizer to invade the red blood cells hence, haemolysis and anaemia. Other symptoms of the disease - e.g., fever, headache, muscle aches, gastrointestinal symptoms, seizures, coma, respiratory distress, and retinopathy - may similarly reflect parasite-induced vitamin A toxicity in the brain and other organs, following the transport of RBCs throughout the body and the release of RA(s) into the circulation (Mawson A et al. Pathogens and global health, 107(3), 122-129). P. falciparum modifies the surface of the infected RBCs and creates an adhesive phenotype, which removes the parasite from circulation for almost half of the asexual life cycle, a unique time frame among the malaria parasites (Milner D et al. Cold Spring Harbor perspectives in medicine, 8(1), a025569).

It was one objective of the present invention to provide a medicament for the treatment diseases caused by a Plasmodium infection, especially for a treatment of malaria.

This objective has been solved by Cytokine-Induced Killer (CIKs) cells that can be used in the treatment of a Plasmodium infection, especially for a Plasmodium falciparum infection, in particular for the treatment of malaria.

The ensuing description provides some embodiment(s) of the invention, and is not intended to limit the scope, applicability or configuration of the invention or inventions. Various changes may be made in the function and arrangement of elements without departing from the scope of the invention as set forth herein.

The terminology used in the description of the disclosure herein is for the purpose of describing particular embodiments only and is not intended to be limiting of the subject matter. As used in this description and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes," "including," "comprises," and/or "comprising," can also be understood as "consisting of" when used in this specification.

CIK cells have been first described by our group in Schmidt-Wolf I. G. H., Negrin R. S., Kiem H., Blume K. G., Weissman I. L. (1991) Use of SCID mouse/human lymphoma modelt o evaluate cytokine-induced killer cells with potent antitumor cell activity. J. Exp. Med., 174(1), 139-149, the disclosure of which is herein fully incorporated by reference.

Cytokine-induced killer cells are CD3-and CD56-positive, non-major histocompatibility complex (MHC)-restricted, natural killer (NK)-like T lymphocytes, generated ex-vivo by incubation of peripheral blood lymphocytes (PBLs) with anti-CD3 monoclonal antibody, interleukin (IL)-2, IL-1, and interferon gamma (IFN-gamma) and then expanded. (see: https://www.cancer.gov/publications/dictionaries/cancer-drug/def/cytokine-inducedkiller-cells)

One potential alternative to reconstitute host immunity is adoptive immunotherapy, which can eliminate cancer cells through transfusing in vitro expanded and activated immune cells, such as CIKs. natural killers (NKs) cytotoxic lymphocytes (CTLs), and tumor-infiltrating lymphocytes (TILs) (Dan Chen et al. Cytokine-induced killer cells as a feasible adoptive immunotherapy for the treatment of lung cancer).

Plasmodium is usually understood as a genus of unicellular eukaryotes that are obligate parasites of vertebrates and insects.

Plasmodium is a member of the phylum Apicomplexa, a large group of parasitic eukaryotes. Within Apicomplexa, Plasmodium is in the order Haemosporida and family Plasmodiidae. Over 200 species of Plasmodium have been described, many of which have been subdivided into 14 subgenera based on parasite morphology and host range.

Species of Plasmodium are distributed globally wherever suitable hosts are found. Insect hosts are most frequently mosquitoes of the genera Culex and Anopheles. Vertebrate hosts include reptiles, birds, and mammals.

The life cycles of Plasmodium species involve development in a blood-feeding insect host, which then injects parasites into a vertebrate host during a blood meal. Parasites grow within a vertebrate body tissue (often the liver) before entering the bloodstream to infect red blood cells. The ensuing destruction of host red blood cells can result in diseases like malaria. During this infection, some parasites are picked up by a blood-feeding insect (mosquitoes in majority cases), continuing the life cycle.

In one embodiment, the Plasmodium infection is selected from infections with Plasmodium falciparum, Plasmodium malariae, Plasmodium ovale, Plasmodium vivax and/or Plasmodium knowlesi, in particular Plasmodium falciparum.

In one embodiment the diseases caused by a Plasmodium infection is malaria.

Malaria is usually understood as a mosquito-borne infectious disease that affects humans and other animals as the mosquito infect the patient with a Plasmodium species. Malaria causes symptoms that typically include fever, tiredness, vomiting, and headaches. In severe cases, it can cause yellow skin, seizures, coma, or death. Symptoms usually begin ten to fifteen days after being bitten by an infected mosquito. If not properly treated, people may have recurrences of the disease months later. In those who have recently survived an infection, reinfection usually causes milder symptoms. This partial resistance disappears over months to years if the person has no continuing exposure to malaria.

As mentioned above, malaria parasites belong to the genus Plasmodium (phylum Apicomplexa). In humans, malaria is usually caused by P. falciparum, P. malariae, P. ovale, P. vivax and P. knowlesi. Among those infected, P. falciparum is the most common species identified (~75%) followed by P. vivax (~20%).

Although P. falciparum traditionally accounts for the majority of deaths, recent evidence suggests that P. vivax malaria is associated with potentially life-threatening conditions about as often as with a diagnosis of P. falciparum infection. P. vivax proportionally is more common outside Africa.

There have been documented human infections with several species of Plasmodium from higher apes. However, except for P. knowlesi these are mostly of limited public health importance.

In one embodiment of the invention the diseases caused by a Plasmodium infection is malaria selected from Malaria tropica, Malaria quartana, Malaria tertiana and/or Malaria quotidiana.

In one embodiment, the invention is characterized in that the CIKs are used for the treatment of diseases caused by a Plasmodium infection is Malaria, in particular selected from Malaria tropica, Malaria quartana, Malaria tertiana and/or Malaria quotidiana, wherein the patient suffering therefrom is resistant against known malaria medications.

Known malaria medications in this context comprise tissue schizontocides, like primaquine, pyrimethamine, sulphonamides and/or proguanil, hypnozoitocides, like primaquine, blood schizontocides, like quinolines such as mepacrine, chloroquine, amodiaquine, mefloquine, quinine, pyronaridine and/or lumefantrine, gametocides, like primaquine, and sporontocides, like primaquine and/or pyrimethamine.

Other known malaria medications in this context comprise Chloroquin, Mefloquin Doxycyclin and/or a mixture of atovaquone/proguanil known under the name malarone.

In one embodiment of the invention, the CIK are derived from eukaryotic cells, preferably Peripheral Blood Mononuclear Cells, that have been treated with at least one of the cytokines IFNy, anti-CD3, IL1-β, IL-2, IL-4, GM-CSF and/or TNFa as described in "CIK cells have been first described by our group in Schmidt-Wolf I. G. H., Negrin R. S., Kiem H., Blume K. G., Weissman I. L. (1991) Use of SCID mouse/human lymphoma modelt o evaluate cytokine-induced killer cells with potent antitumor cell activity. J. Exp. Med., 174(1), 139-149)".

In one embodiment of the invention, the CIKs are CD3- and/or CD56-positive, preferably CD-3 positive.

In one embodiment of the invention, the CIK are derived from eukaryotic cells, preferably Peripheral Blood Mononuclear Cells, that have been treated with IFNy, anti-CD3, IL1 and/or IL-2.

In one embodiment of the invention, the CIK are derived from eukaryotic cells, preferably Peripheral Blood Mononuclear Cells, that have been treated with at least one of the cytokines IFNy, anti-CD3, IL-1β and/or IL-2.

In one embodiment of the invention, the CIK are derived from eukaryotic cells, preferably Peripheral Blood Mononuclear Cells, that have been treated with IFN-γ, IL-1β, IL-2 and anti-CD3 in that order.

In one embodiment of the invention, the CIK are derived from eukaryotic cells, preferably Peripheral Blood Mononuclear Cells, that have been treated with IFN-γ on day 1, IL-1β on day 2, IL-2 on day 2 and anti-CD3 on day 2.

In one embodiment of the invention, the CIK are derived from eukaryotic cells, preferably Peripheral Blood Mononuclear Cells, that have been treated with IFN-γ on day 1, IL-1β on day 2, IL-2 on day 2 and anti-CD3 on day 2, wherein 11-2 is further added on day 3 and continuously after every 3 days.

The present invention is now further illustrated in the following examples. The examples should not be considered as limiting the invention in any way.

Examples

General reagents:

CIK Medium preparation:

- RPMI 1640: remove 67.5ml, then add:

    12.5 mL 4-(2-hydroxyethyl)-1-piperazineethanesulfonic acid (HEPES) buffer
    1% (5 mL) of Penicillin/Streptomycin solution (P/S)
    10% (50 mL) of fetal bovine serum (FBS) (aliquot)

**Other solution preparation:**

- PBS-EDTA solution (1:250): 500ml PBS + 2ml EDTA (0.5M, 500ml)

- Erythrocyte lysis buffer: 1ml erythrocyte lysis stock+ 9ml sterile distilled water

- IFN-$\gamma$: ImmunoTools (cat. 11343536, 100 $\mu$g): final concentration is 1000U/ml. + 1ml distilled water to obtain 50 aliquots (2000U/ $\mu$L, 20 $\mu$L, 20 $\mu$L/tube)

- IL-1$\beta$: Your ImmunoTools-Team company (cat.No.11340013, 10 $\mu$g), the final concentration is 100U/ml. 50 aliquots (20000 U/$\mu$L, 2 $\mu$L, 10 tubes) made with 100 $\mu$L sterile distilled water, then sub-aliquoted (40U/ $\mu$L, 1000 $\mu$L, 100 $\mu$L /tube) with 1ml distilled water when used for CIK culture.

- Anti-CD3: 2$\mu$L of the stock (1mg/mL), no further dilution.

- IL-2: ImmunoTools-Team (cat.No.11340027, 250 $\mu$g), the final concentration is 600U/ml, 100 aliquots (1000 U/$\mu$L, 25 $\mu$L, 24$\mu$L/test) made with 2.5ml distilled water.

General methods:

    1. Blood Collection: Citrated blood group A+ is provided by the Hematology unit of the University Teaching hospital Bonn. Blood is centrifuged at 2000 rpm for 5 minutes to separate serum and buffy coat from red blood cells (RBCs). RBCs and buffy coats are further purified for Plasmodium culture and CIK cells generation, respectively.

    2. CIK Cells Generation : The procedure developed by the Med. 1 laboratory of AG Schmidt-Wolf of the University hospital, Bonn is used. PBMCs are treated for at least 14 days with cytokines. Maturation of CIK cells is analyzed using flow cytometry by checking surface expression of CD3, CD56 and CD8.
    In particular, CIK cells were generated as follows:

    1- Pipette 25 mL of PBS-EDTA solution (1:250) to a Falcon tube.
    2- Add 25 mL of Blood (1:1 ratio) and mix gently.
    3- In another Falcon tube pipette 15 mL of Ficoll.
    4- Take 15 mL of the blood-PBS solution, and start pipetting slowly, make sure the setting in the pipette is set on "Ex".
    5- Repeat step 4, in the end the tube should be filled up to 45 mL mark, the lower 15 mL are visibly separated from the upper 30 mL of blood.

6- Centrifuge at 1000 rpm for 30 min (this will last for a total of 1 hour)

7- Take the tube out of the centrifuge gently, Avoid shaking it!

8- With a smaller pipette (5-10 mL), suck the white layer, or the buffy coat and transfer to a new 15 mL PBS/EDTA-containing tube.

9- Fill the tube up to 50 mL with PBS/EDTA and centrifuge at 800 rpm for 7 min.

10- Discard the supernatant, and add 10 mL of erythrocyte lysis buffer. Resuspend the pellet very well, and place on ice for 10 min.

11- Repeat step (9).

12- Discard the supernatant, and Resuspend the pellet in 5 mL of CIK cell medium

13- Dilute 100 μL of the cell suspension with 900 μL of PBS or Medium.

14- Take 10 μL of the diluted suspension and add it to 90 μL of trypan blue dye.

15- Take 10 μL of the solution, and count under microscope using Neubauer Chamber.

16- Calculate the total cell count as follows:

$$\frac{ce\ coun\ x\ 10\ x\ 10\ x\ 5}{4}\ X\ 10^4 = \text{total cell count in 5 mL}$$

17- Day 0: Seed $75x10^6$ cells in 40 mL of CIK media in T-175 cell culture flask.

18- Wait at least 2 hours and transfer all the medium to a new T-175 flask. Add 20 μL of IFN-γ (2000 U/μL).

19- Next day add 100 μL of IL-1β (40U/μL per subaliquot, 1000μL) and 2 μL anti-CD3 (1mg/mL), as well as 24 μL of IL-2 (1000 U/μL).

20- Continue to add Il-2 on day 3 and after every 3days.

3. Culture of Plasmodium falciparum: This is achieved following the 3D7 technique employed by the Microbiology laboratory of the University Hospital, Bonn. In this method, the parasites are cultured by infecting purified RBC in malaria culture medium (MCM = RPMI+ Gentamycin+ Albumax+ Hypoxanthin) at 37°C in a gaseous atmosphere (3% Oxygen, 5% Carbondioxide and 92% Nitrogen). Plasmodium continuous culture is maintained by adding non-infected RBCs to keep parasitaemia level between 1-6%. For every second intra-erythocytic life-cycle a synchronization by single sorbitol treatment is performed. Parasitemia level is measured using Giemsa stain.

4. Giemsa Staining and Microscopy : Cultured blood smear slide is fixed in ethanol for 1 minute, stained with Giemsa stain (working solution = 1 ml Giemsas Azur Eosin-Methylene blue + 19 ml buffer solution) for 12-15 minutes, rinsed with distilled water and air-dried. Counting of the parasites (trophozoites and schizonts) is done with the light microscope at magnification 100 X (oil immersion).

5. Plasmodium and CIK cells Co-culture : E/T ratios for the co-culture is determined after the parasitemia has been done. Cytotoxicity is investigated by measuring the remaining parasitaemia level with Giemsa staining.

Co-culture of CIK Cells and Plasmodium falciparum (Pf) infected erythrocytes (RBCs).

1. After centrifuging 13 days old CIK cells and the supernatant discarded, they were diluted with 1ml of CIK cell medium.

2. 10 μl was aliquoted and stained with 90 μl of trypan blue and counted using Neubauer counter.

3. 100 μl from (1) was then aliquoted for phenotyping using flow cytometry (see Figure .

4. The supernatant from the Pf culture plate was sucked off and thin smear slide of the RBCs was prepared and air-dried.

5. The slide was fixed with acetone for 30 seconds and stained with 1:10 dilution of Giemsa for 10 minutes, after which it was washed with running water and dried.

6. Parasite counting was done at immersion oil magnification (100X) of a light microscope and quantitation done in percentage.

7. In a 96well culture plate, 160 $\mu$l of malaria culture medium (MCM= RPMI+ gentamycin+ albumax+ hypoxanthin)) was filled in each of the first three wells.

8. Control wells (4 and 5) were filled with 100 $\mu$l of the MCM each.

9. Into wells 1-3, 20 $\mu$l of CIK cells was added into each, and 100ul added to well 4.

10. 20 $\mu$l of RBCs were put into well 1, 30ul into well 2, 10ul into well 3 and 100ul into well 5.

11. All mixtures were mixed by gently pipetting up and down.

12. The plate was then slantly covered, placed in a gas chamber, chamber filled with gas (3% Oxygen, 5% Carbon dioxide and 92% Nitrogen) and then incubated at 37°C for 24 hours.

13. Thin smear slides were prepared from wells 1-3 and 5, then same procedure of (5) and (6) performed.

14. 10 $\mu$l from well 4 was again stained with trypan blue to check that the CIK cells were not lysed.

Results:

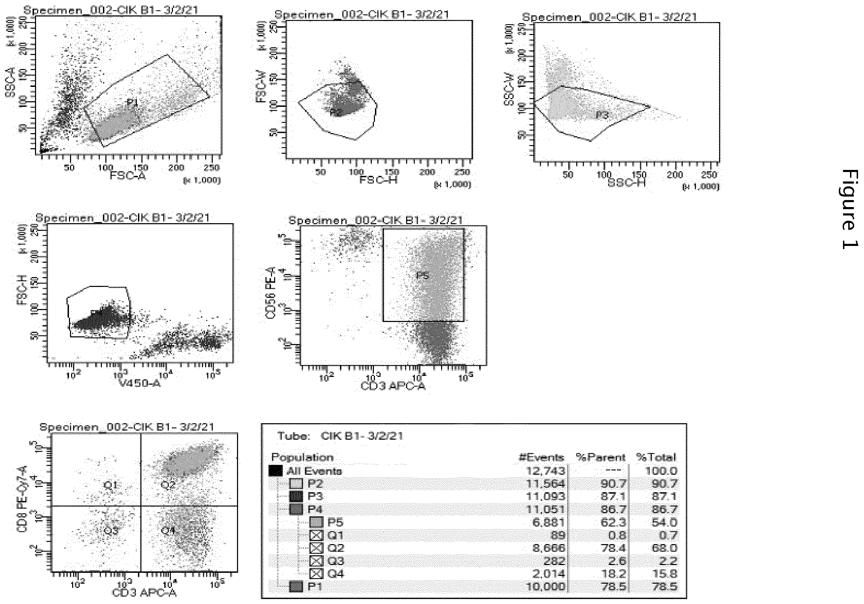

Characterization of the CIK cells is done based on the phenotypic expression of CD3 marker, as seen in figure 1. Figure 1 shows a good expression of CD3 positive cells (quadrant 4) on FACS.

## Number of CIK cells counted = $51*10^6$ cells/ml

1million cells ~20 $\mu$l
Parasitaemia before co-culture = 2.9% (29 parasites counted in 1000 RBCs)
Parasitaemia after co-culture as seen in table 1 below

|  | Vol. of CIK cells / $\mu$l | Vol. of RBCs / $\mu$l | Vol. of medium/ $\mu$l | Parasitemia before co-culture / % | Parasitemia aftr co-culture / % |
|---|---|---|---|---|---|
| Well 1 | 20.0 | 20.0 | 160.0 | 2.9 | 2.0 |
| Well 2 | 20.0 | 30.0 | 160.0 | 2.9 | 2.2 |
| Well 3 | 20.0 | 10.0 | 160.0 | 2.9 | 0.6 |
| Well 4 (control) | 100 | --- | 100 | Normal | Normal |
| Well 5 (control) | --- | 100 | 100 | 2.9 | 4.0 |

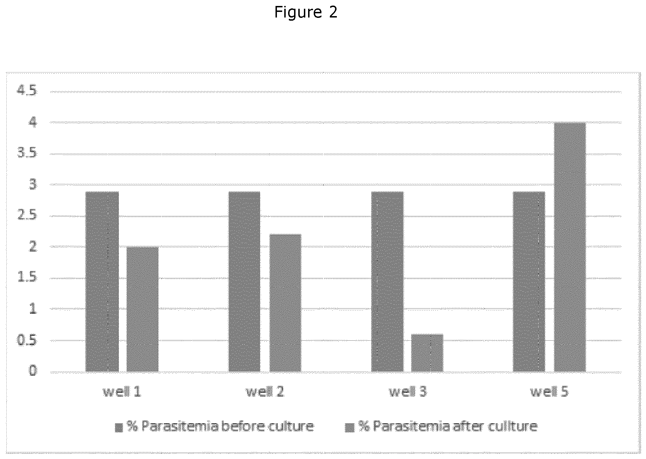

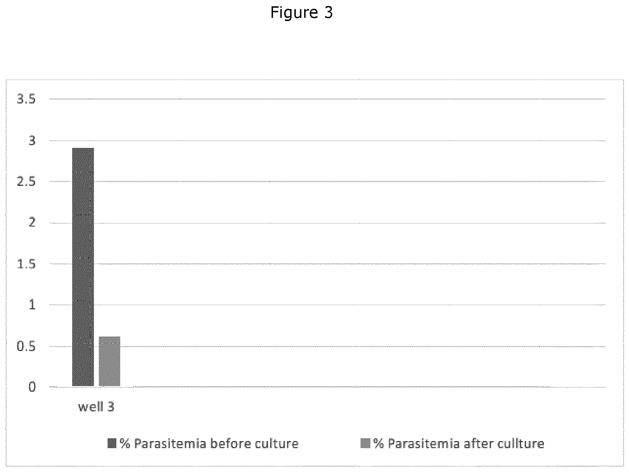

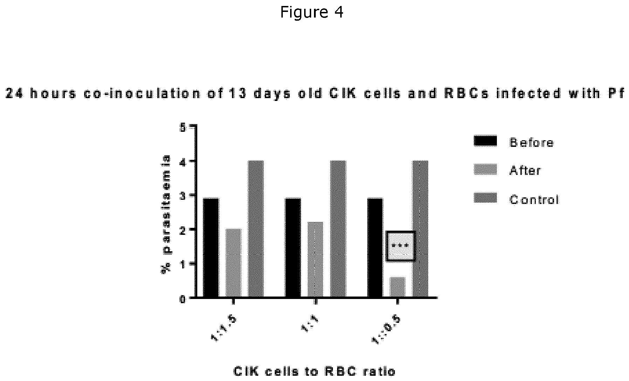

A comparison of 24 hours co-inoculation of 13 days old CIK cells and RBCs infected with Pf is given in Figure 2.
A comparison of Well 3 showing a 79.3% decrease of parasitaemia after co-culture (CIK cells to RBC ratio= 2:1) is given in Figure 3.
In a further experiment, CIK were co-cultured with Pf in RBCs for 24 hrs at CIK cells to RBCs ratios of 1:1.5, 1:1 and 1:0.5 in a 96 well culture plate.
The results are given in Figure 4.

## The % parasitaemia = number of parasites counted against 1000 RBCs*100.

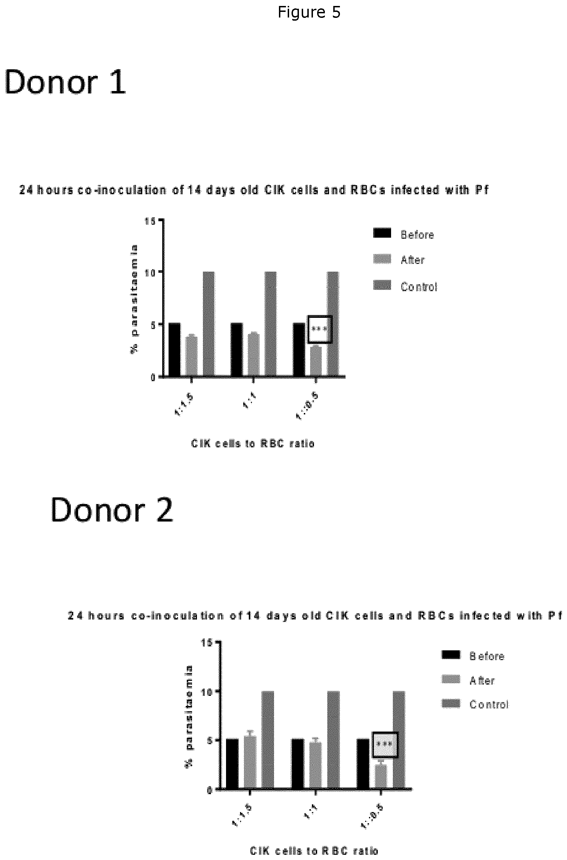

The marked parasitaemia decreased for 85% at a ratio of 1:0.5.
The same experiment was repeated with cells from two other donors (D1 and D2).
The marked parasitaemia decreased for 71.7% and 74.3% for D1 and d2, respectively.
The results are given in Figure 5.

**Claims**

1. Cytokine induced Killer cells (CIKs) for use in the treatment of diseases caused by a Plasmodium infection.

2. CIKs for use according to claim 1, wherein the Plasmodium infection is selected from infections with Plasmodium falciparum, Plasmodium malariae, Plasmodium ovale, Plasmodium vivax and/or Plasmodium knowlesi.

3. CIKs for use according to any of the preceding claims, wherein the diseases caused by a Plasmodium infection is Malaria, in particular selected from Malaria tropica, Malaria quartana, Malaria tertiana and/or Malaria quotidiana.

4. CIKs for use according to any of the preceding claims, wherein the diseases caused by a Plasmodium infection is Malaria, in particular selected from Malaria tropica, Malaria quartana, Malaria tertiana and/or Malaria quotidiana and wherein the patient suffering therefrom is resistant against known malaria medications.

5. CIKs for use according to any of the preceding claims, wherein the CIKs are CD3- and/or CD56-positive, preferably CD-3 positive.

6. CIKs for use according to any of the preceding claims, wherein the CIKs are derived from eukaryotic cells, preferably Peripheral Blood Mononuclear Cells, that have been treated with at least one of the cytokines IFNy, anti-CD3, IL-1β and/or IL-2.

7. CIKs for use according to any of the preceding claims, wherein the CIKs are derived from eukaryotic cells, preferably Peripheral Blood Mononuclear Cells, that have been treated with IFN-γ, IL-1β, IL-2 and anti-CD3 in that order.

8. CIKs for use according to any of the preceding claims, wherein the CIKs are derived from eukaryotic cells, preferably Peripheral Blood Mononuclear Cells, that have been treated with IFN-γ on day 1, IL-1β on day 2, IL-2 on day 2 and anti-CD3 (1mg/mL) on day 2.

9. CIKs for use according to any of the preceding claims, wherein the CIK are derived from eukaryotic cells, preferably Peripheral Blood Mononuclear Cells, that have been treated with IFN-γ on day 1, IL-1β on day 2, IL-2 on day 2 and anti-CD3 on day 2, wherein Il-2 is further added on day 3 and continuously after every 3 days.

Figure 1

Figure 2

Figure 3

Figure 4

**24 hours co-inoculation of 13 days old CIK cells and RBCs infected with Pf**

Figure 5

# Donor 1

**24 hours co-inoculation of 14 days old CIK cells and RBCs infected with Pf**

# Donor 2

**24 hours co-inoculation of 14 days old CIK cells and RBCs infected with Pf**

Europäisches
Patentamt

European
Patent Office

Office européen
des brevets

## EUROPEAN SEARCH REPORT

Application Number

EP 21 16 3132

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SCHMIDT-WOLF I G H ET AL: "USE OF A SCID MOUSE/HUMAN LYMPHOMA MODEL TO EVALUATE CYTOKINE-INDUCED KILLER CELLS WITH POTENT ANTITUMOR CELL ACTIVITY", JOURNAL OF EXPERIMENTAL MEDICINE, ROCKEFELLER UNIVERSITY PRESS, US, vol. 174, no. 1, 1 July 1991 (1991-07-01), pages 139-149, XP001182112, ISSN: 0022-1007, DOI: 10.1084/JEM.174.1.139 * abstract * * page 140, left-hand column * ----- | 1-9 | INV. A61P33/06 A61K35/17 C12N5/0783 |
| A | ZHANG YING ET AL: "Ten-year update of the international registry on cytokine-induced killer cells in cancer immunotherapy", JOURNAL OF CELLULAR PHYSIOLOGY, vol. 235, no. 12, 1 December 2020 (2020-12-01), pages 9291-9303, XP055809744, US ISSN: 0021-9541, DOI: 10.1002/jcp.29827 * the whole document * ----- | 1-9 | |
| A | Stefan Gütgemann ET AL: "Cytokine-induced killer cells are type II natural killer T cells", German medical science : GMS e-journal, 10 September 2007 (2007-09-10), pages Doc07-Doc07, XP055619025, Germany Retrieved from the Internet: URL:https://www.ncbi.nlm.nih.gov/pmc/articles/PMC2703238/pdf/GMS-05-07.pdf * the whole document * ----- | 1-9 | TECHNICAL FIELDS SEARCHED (IPC) C12N A61K A61P |

-/--

The present search report has been drawn up for all claims

| Place of search | Date of completion of the search | Examiner |
|---|---|---|
| Munich | 16 September 2021 | Domingues, Helena |

EPO FORM 1503 03.82 (P04C01)

page 1 of 3

Europäisches
Patentamt

European
Patent Office

Office européen
des brevets

**EUROPEAN SEARCH REPORT**

Application Number

EP 21 16 3132

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Xingchun Gao,Yajing Mi, Na Guo, Hao Xu, Lixian Xu, Xingchun Gou, and Weilin Jin: "Cytokine-Induced Killer Cells As Pharmacological Tools for Cancer Immunotherapy", , 1 January 2017 (2017-01-01), XP055593528, Retrieved from the Internet: URL:https://www.ncbi.nlm.nih.gov/pmc/articles/PMC5498561/ [retrieved on 2019-06-03] * the whole document * | 1-9 | |
| A | CN 109 078 180 A (XINFU BEIJING PHARMACEUTICAL TECH CO LTD) 25 December 2018 (2018-12-25) * claims 8,33 * | 1-5 | |
| A | CHEN Q. ET AL: "Human natural killer cells control Plasmodium falciparum infection by eliminating infected red blood cells", PROCEEDINGS OF THE NATIONAL ACADEMY OF SCIENCES, vol. 111, no. 4, 13 January 2014 (2014-01-13), pages 1479-1484, XP055841613, US ISSN: 0027-8424, DOI: 10.1073/pnas.1323318111 * the whole document * | 1-9 | TECHNICAL FIELDS SEARCHED (IPC) |

-/--

The present search report has been drawn up for all claims

| Place of search | Date of completion of the search | Examiner |
|---|---|---|
| Munich | 16 September 2021 | Domingues, Helena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 21 16 3132

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BURRACK KRISTINA S. ET AL: "Contributions of natural killer cells to the immune response against Plasmodium", MALARIA JOURNAL, vol. 18, no. 1, 18 September 2019 (2019-09-18), XP055841615, DOI: 10.1186/s12936-019-2953-1 Retrieved from the Internet: URL:http://link.springer.com/article/10.1186/s12936-019-2953-1/fulltext.html> * the whole document * ----- | 1-9 | |

TECHNICAL FIELDS
SEARCHED (IPC)

The present search report has been drawn up for all claims

| Place of search | Date of completion of the search | Examiner |
|---|---|---|
| Munich | 16 September 2021 | Domingues, Helena |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 3 of 3

## ANNEX TO THE EUROPEAN SEARCH REPORT
## ON EUROPEAN PATENT APPLICATION NO.

EP 21 16 3132

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2021

| Patent document cited in search report | Publication date | Patent family member(s) | Publication date |
|---|---|---|---|
| CN 109078180 A | 25-12-2018 | NONE | |

EPO FORM P0459

For more details about this annex : see Official Journal of the European Patent Office, No. 12/82

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Non-patent literature cited in the description

- **DEIRDRE et al.** *Science,* 11 April 2003, vol. 300 (5617), 318-21
- **AAYUSH KHADKA et al.** *Malar J,* 2018, vol. 17, 224
- **VYGEN-BONNET et al.** *Changes in malaria epidemiology in Germany,* 2001, https://www.ecdc.europa.eu/en/publications-data/malaria-annual-epidemiologicalreport-2018
- **MAWSON A et al.** *Pathogens and global health,* vol. 107 (3), 122-129
- **MILNER D et al.** *Cold Spring Harbor perspectives in medicine,* vol. 8 (1), a025569
- **SCHMIDT-WOLF I. G. H. ; NEGRIN R. S. ; KIEM H. ; BLUME K. G. ; WEISSMAN I. L.** Use of SCID mouse/human lymphoma modelt o evaluate cytokine-induced killer cells with potent antitumor cell activity. *J. Exp. Med.,* 1991, vol. 174 (1), 139-149